(19) Europäisches Patentamt
European Patent Office
Office européen des brevets

(11) **EP 3 116 644 B1**

(12) **EUROPÄISCHE PATENTSCHRIFT**

(45) Veröffentlichungstag und Bekanntmachung des Hinweises auf die Patenterteilung:
**27.02.2019 Patentblatt 2019/09**

(21) Anmeldenummer: **15709944.1**

(22) Anmeldetag: **13.03.2015**

(51) Int Cl.:
*B01J 20/32* *(2006.01)*      *A61M 1/36* *(2006.01)*
*B01J 20/28* *(2006.01)*

(86) Internationale Anmeldenummer:
**PCT/EP2015/055368**

(87) Internationale Veröffentlichungsnummer:
**WO 2015/136107 (17.09.2015 Gazette 2015/37)**

(54) **ADSORBERGRANULAT ZUR ENTFERNUNG URÄMISCHER TOXINE**

GRANULAR ADSORBENTS FOR REMOVAL OF UREMIC TOXINS

ADSORBANTS GRANULAIRES POUR L'ÉLIMINATION DE TOXINES URÉMIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.03.2014 EP 14159881**

(43) Veröffentlichungstag der Anmeldung:
**18.01.2017 Patentblatt 2017/03**

(73) Patentinhaber:
• **Fresenius Medical Care Deutschland GmbH**
**61352 Bad Homburg (DE)**
• **PSS Polymer Standards Service GmbH**
**55120 Mainz (DE)**

(72) Erfinder:
• **TSCHULENA, Ulrich**
**61352 Bad Homburg (DE)**
• **REINHOLD, Günter**
**55120 Mainz (DE)**
• **PASSLICK-DEETJEN, Jutta**
**77960 Seelbach (DE)**
• **LEINENBACH, Hans Peter**
**A-3500 Krems (AT)**
• **HÖRBERG, Annika**
**55120 Mainz (DE)**
• **OLESCHKO, Kirsten**
**55120 Mainz (DE)**

(74) Vertreter: **Breuninger, Marcus**
**Fresenius Medical Care**
**AG & Co. KGaA**
**Global Patents & IP**
**Else-Kröner-Strasse 1**
**61352 Bad Homburg (DE)**

(56) Entgegenhaltungen:
EP-A1- 0 069 435     WO-A1-2004/060554
WO-A1-2006/002151     FR-A1- 2 148 022
FR-A1- 2 239 282     GB-A- 1 562 969
US-A- 4 140 652

**Beschreibung**

**Technisches Gebiet**

Die vorliegende Erfindung betrifft hämokompatible Adsorbermaterialien zur Entfernung urämischer Toxine, das Verfahren zur Herstellung der Adsorbermaterialien und deren Verwendung in der Nieren- oder Lebererersatztherapie, sowie zur Entgiftung und zur Behandlung einer Sepsis.

Die Aufgabe der gesunden Niere ist die Ausscheidung von Endprodukten des Stoffwechsels (harnpflichtige Substanzen) und Giftstoffen (Urämietoxine) aus dem Körper durch Bildung des Harns. Die Niere entfernt dabei ein breites Spektrum an Substanzen unterschiedlichen Molekulargewichts. Eine Übersicht über urämische Toxine wurde von R. Vanholder et al. veröffentlicht. (R. Vanholder et al., Kidney International, 63 (2003) 1934 - 1943) Die urämischen Toxine werden an Hand ihres Molekulargewichts in drei Klassen eingeteilt. Toxine mit einem Molekulargewicht von unter 500 Dalton bilden die Gruppe mit niedrigem Molekulargewicht. Die Mittelmoleküle liegen in einem mittleren Bereich mit einem Molekulargewicht zwischen 500 und 12 000 D. Zu den Mittelmolekülen gehört beispielsweise beta2-Microglobulin (11800 D). Die dritte Klasse von Urämietoxinen bilden Moleküle mit einem Molekulargewicht von über 12 000 D.

Darüber hinaus wird nach der Wasserlöslichkeit der Urämietoxine unterschieden. Beispiele für gut wasserlösliche urämische Toxine mit einem niedrigen Molekulargewicht sind Harnstoff, Creatinin, Oxalate, Guanidine und Harnsäure.

Beispiele für schlecht wasserlösliche urämische Toxine sind p-Cresol, Indoxylsulfat, Phenol, Hippursäure und Homocystein. Diese Urämietoxine liegen im Serum überwiegend an Proteinen gebunden vor.

Bei gesunden Personen werden die Urämietoxine über die Niere mit dem Harn ausgeschieden. Beim chronischen Nierenversagen verbleiben die urämischen Toxine jedoch im Blut des Patienten und müssen durch Hämodialyse oder Peritonealdialyse entfernt werden.

Während die Entfernung wasserlöslicher Toxine wie beispielsweise Harnstoff oder Creatinin mittels Hämodialyse sehr gut möglich ist, ist die Entfernung von schlecht wasserlöslichen hydrophoben Urämietoxinen mittels Hämodialyseverfahren durch die Proteinbindung stark erschwert. Man geht allgemein davon aus, dass ein chemisches Gleichgewicht zwischen dem freien, gelösten Toxin und dem proteingebundenen Toxin vorliegt, das weit auf Seiten des proteingebundenen Toxins liegt. Dies bedeutet, dass der überwiegende Teil dieser Urämietoxine an Proteine gebunden ist und nur ein kleiner Teil im Blutplasma gelöst ist.

Da es sich bei einem großen Teil der Substanzen um niedermolekulare Komponenten handelt, die zu einem geringen Teil in freier Form vorliegen, sind sie prinzipiell dialysierbar.

Es gibt verschiedene Serumproteine, wie z.B. Albumin die als Bindungspartner der hydrophoben Urämietoxine fungieren. Die Serumproteine werden auf Grund ihres Molekulargewichtes von Dialysemembranen zurückgehalten. Die Serumproteine werden durch Hämodialyseverfahren also nicht entfernt. Damit kann nur der freie, gelöste Anteil der urämischen Toxine aus dem Blut des Patienten entfernt werden.

Die Einstellung des Gleichgewichtes unter der Dialyse wird zum geschwindigkeitsbestimmenden Schritt. Es ist zwar zu erwarten, dass sich nach der Entfernung der gelösten Toxine aus dem Blut das Gleichgewicht zwischen freien und proteingebundenen Toxinen wieder neu einstellt und bei genügend langer Dialysezeit ein beträchtlicher Teil der Toxine entfernen lässt, diese Zeit steht bei Hämodialysebehandlungen jedoch nicht zur Verfügung.

Die Konzentration von proteingebundenen Toxinen im Plasma von Patienten mit chronischem Nierenversagen ist im Vergleich zu nierengesunden Patienten stark erhöht. Es konnte gezeigt werden, dass die Konzentration von proteingebundenen urämischen Toxinen, wie beispielsweise Indoxylsulfat oder auch para-Cresylsulfat korreliert ist mit der Mortalität von Dialysepatienten. Patienten mit einer hohen Konzentration dieser Toxine zeigten eine verstärkte Mortalität (Liabeuf et al., Nephrol Dial Transplant 25 (2010) 1183-1191; Barreto et al., Clin JAm Soc Nephrol 4 (2009) 1551-1558,).

Es besteht also ein erheblicher Bedarf an therapeutischen Verfahren, welche die proteingebundenen urämischen Toxine effektiv aus dem Blut des Patienten entfernen.

WO 2013/004604 offenbart ein Verfahren zur Entfernung proteingebundener Toxine mit Hilfe hochfrequenter, elektromagnetischer Felder. Unter dem Einfluss eines hochfrequenten, elektromagnetischen Feldes werden die urämischen Toxine aus der Proteinbindung freigesetzt und können mittels Dialyse entfernt werden.

Eine weitere Möglichkeit zur Entfernung von Toxinen aus Blut oder Blutplasma ist die Bindung der Toxine an einen hydrophoben Adsorber. Aus dem Stand der Technik sind solche Adsorber bekannt. Die zur Entfernung von Giftstoffen aus Blut verwendeten Adsorber bestehen aus Aktivkohle oder Styrol-Divinylbenzol Copolymer.

Ein Problem dieses Adsorbermaterials ist jedoch die unzureichende Hämokompatibilität. Kommt Blut mit der Oberfläche des Adsorbermaterials in Kontakt so können zwei Probleme auftreten. Zum einen kann feinteiliger Abrieb in den Organismus gelangen und zu Embolien führen, zum anderen kann die hydrophobe Oberfläche der Aktivkohle zur Blutgerinnung und Komplementaktivierung führen.

US 3983053 offenbart mit Polymeren überzogenes Adsorbermaterial.

DE 7425290 U offenbart eine Blutdurchflusskolonne zum Entgiften von Blut, die mit einem teilchenförmigen Absorbermittel gefüllt ist.

US 4048064 offenbart ein biokompatibles System zur Hämoperfusion umfassend ein polymerbeschichtetes Adsorbermaterial.

In US 4169051 wird ein Verfahren zur Blutreinigung an mit Nitrocellulose beschichteten Aktivkohlekügelchen beschrieben.

US 2004/001800 beschreibt ein Adsorbermaterial bestehend aus einem porösen, hydrophoben Polymerkern und einer hydrophilen Beschichtung. Die Synthese der Polymerkerne aus Divinylbenzol und Ethylvinylbenzol und die Beschichtung erfolgt in zwei Stufen in einem Reaktor. FR 2148022 offenbart ein Adsorbermaterial bestehend aus einem Aktivkohlepartikel und einer quervernetzten Polymerschicht aus Polyvinylpyrrolidon.

FR 2239282 offenbart Sorptionsmaterialen, welche mit quervernetzten hydrophilen Polymeren beschichtet sind.

Die im Stand der Technik beschriebenen Adsorber finden Anwendung in der Entfernung von Toxinen aus dem Blut von Patienten mit akutem Leberversagen oder einer Vergiftung.

Bei den aus dem Stand der Technik bekannten Aktivkohleadsorbern wird die Polymerbeschichtung folgendermaßen aufgebracht. Ein Polymer wird in einem geeigneten organischen Lösungsmittel gelöst, die Aktivkohleträger in dieser Lösung suspendiert und das Lösungsmittel entfernt. Optional wird das abgeschiedene Polymer zuvor noch quervernetzt. Die vollständige Entfernung der verwendeten Lösungsmittel ist wegen deren Toxizität unerlässlich.

Es besteht daher ein Bedarf an einem hämokompatiblen Adsorbern zur Adsorption proteingebundener urämischer Toxine.

## Beschreibung der Erfindung

Die vorliegende Erfindung betrifft hämokompatible Adsorbermaterialien zur Entfernung urämischer Toxine mit den Merkmalen des Oberbegriffs des Anspruchs 1.

Die vorliegende Erfindung betrifft insbesondere ein hämokompatibles Adsorbergranulat zur Entfernung proteingebundener Toxine aus Blut oder Blutplasma umfassend sphärische Aktivkohlepartikel, die mit einer hydrophilen Polymerschicht überzogen sind. Die Polymerschicht ist quervernetzt.

In der vorliegenden Erfindung umfasst die hydrophile Polymerschicht Polyvinylpyrrolidon (PVP).

Es wurde gefunden, dass die Hämokompatibilität des beschichteten Adsorbergranulats mit der Schichtdicke des hydrophilen Polymers zunimmt. In einer bevorzugten Ausführungsform der vorliegenden Erfindung beträgt die Schichtdicke der hydrophilen Polymerschicht auf dem Adsorbergranulat ungefähr 50 nm bis 20 $\mu$m, bevorzugt 0,1 $\mu$m bis 15 $\mu$m, besonders bevorzugt 0,5 $\mu$m bis 10 $\mu$m.

Die Form des Adsorbergranulates hat einen entscheidenden Einfluss auf die Hämokompatibilität. Es wurde gefunden, dass die Hämokompatibilität einer von Vollblut durchströmten Adsorberschüttung zunimmt je kugelförmiger die einzelnen Adsorberpartikel sind. Das heißt, mit zunehmender Sphärizität des Adsorbermaterials steigt auch die Biokompatibilität. Die Sphärizität ist eine Kenngröße dafür, wie kugelförmig ein Körper ist. Die Sphärizität $\Psi$ eines Körpers K ist das Verhältnis der Oberfläche einer Kugel gleichen Volumens zur Oberfläche des Körpers:

$$\Psi = \frac{\pi^{1/3}(6V_P)^{2/3}}{A_P} \ .$$

Wobei Vp das Volumen des Körpers und $A_P$ seine Oberfläche bezeichnet.

Das erfindungsgemäße Adsorbergranulat besitzt eine Sphärizität von größer als 0,9, bevorzugt größer 0,92, besonders bevorzugt größer als 0,95.

Neben der Form des Adsorbergranulates hat auch der mittlere Durchmesser einen entscheidenden Einfluss auf die Hämokompatibilität. Eine Schüttung aus Partikeln übt gegenüber dem sie umströmenden Fluid eine Widerstandskraft aus. Dies führt zu einem Druckverlust über der Schüttung. Je kleiner die Partikel werden umso größer wird der resultierende Druckverlust. Daraus resultiert eine geringere Hämokompatibilität bei kleiner werdenden Partikeldurchmessern.

Andererseits beeinflusst die Oberfläche der Partikel die Adsorptionsgeschwindigkeit. Da das Volumen einer Kugel mit der dritten Potenz ihres Radius wächst, die Oberfläche jedoch nur mit der zweiten Potenz, sollte der mittlere Durchmesser des Adsorbergranulates nicht zu groß sein, um die Adsorptionseigenschaften nicht negativ zu beeinflussen.

Das erfindungsgemäße Adsorbergranulat umfasst ein Aktivkohlegranulat, das eine mittlere Partikelgröße von 100 $\mu$m bis 1000 $\mu$m aufweist. In einer besonders bevorzugten Ausführungsform beträgt die mittlere Partikelgröße 200

- 600 μm.

**Kurze Beschreibung der Figuren**

Figur 1 zeigt die Plasmakonzentration des TAT-III-Komplexes von Vollblut nach Kontakt mit Adsorbergranulaten der Beispiele 1 bis 4 im Vergleich zu Granulaten aus einem DALI-Adsorber und aus einer Kartusche vom Typ Adsorba 300C.

Figur 2 zeigt die Plasmakonzentration Komplementkomponente C5a von Vollblut nach Kontakt mit Adsorbergranulaten der Beispiele 1 bis 4 im Vergleich zu Granulaten aus einem DALI-Adsorber und aus einer Kartusche vom Typ Adsorba 300C.

Figur 3 zeigt die Leukozytenzahl (als Prozent vom Ausgangswert) in Vollblut nach 180 min Kontakt mit Adsorbergranulaten der Beispiele 1 bis 4 im Vergleich zu Granulaten aus einem DALI-Adsorber und aus einer Kartusche vom Typ Adsorba 300C.

Figur 4 zeigt die Thrombozytenzahl (als Prozent vom Ausgangswert) in Vollblut nach 180 min Kontakt mit Adsorbergranulaten der Beispiele 1 bis 4 im Vergleich zu Granulaten aus einem DALI-Adsorber und aus einer Kartusche vom Typ Adsorba 300C.

Die Porengrößenverteilung der beschichteten Granulate kann durch die Wahl der Reaktionstemperatur beeinflusst werden. Die Zerfallsgeschwindigkeit des Radikalstarters hängt von der Reaktionstemperatur ab. Bei höheren Reaktionstemperaturen bilden sich mehr Radikale und dies führt zu einer engeren Porengröße. Zusätzlich wird die Porengrößenverteilung durch die Polymermenge und die Vernetzermenge beeinflusst. Die Porengröße hat einen wichtigen Einfluss auf die Selektivität der adsorbierten Toxine. Durch eine kleine Porengröße kann die Bindung von Proteinen oder anderen Makromolekülen, wie z.B. Albumin oder Heparin, verhindert werden. Dadurch kann die Hämokompatibilität verbessert werden. Zusätzlich wird verhindert, dass gewünschte Substanzen, wie z.B. Albumin durch einen Adsorber aus dem Blut entfernt werden.

Der Reaktionsmischung können Stabilisatoren zugesetzt werden, die verhindern sollen, dass die entstehenden beschichteten Partikel zusammenbacken. Als Stabilisatoren eignen sich beispielsweise Cellulosederivate, Polyvinylalkohole, Natriumlaurylsulfat (SDS, SLS) oder Polyvinylpyrrolidon (PVP). In einer bevorzugten Ausführungsform der vorliegenden Erfindung ist der Stabilisator Polyvinylpyrrolidon, besonders bevorzugt in Form einer 2 %-igen wässrigen Lösung.

Der Reaktionsmischung werden weiterhin Quervernetzer zugegeben. Durch Quervernetzung erhöhen sich die Härte und die Zähigkeit der Polymerbeschichtung. Weiterhin nimmt die Löslichkeit der Schicht ab. In der vorliegenden Erfindung wird die hydrophile Polymerschicht mit Ethylenglykoldimethacrylat quervernetzt.

Durch Variation des Stoffmengenverhältnisses von hydrophilem Monomer zu Quervernetzer können die Eigenschaften der Polymerschicht beeinflusst werden. In einer bevorzugten Ausführungsform der vorliegenden Erfindung beträgt das Stoffmengenverhältnis von Monomer zu Quervernetzer größer als 68 : 32, vorzugsweise zwischen etwa 80 : 20 und etwa 95 : 5, besonders bevorzugt zwischen etwa 80 : 20 und etwa 90 : 10.

Die vorliegende Erfindung betrifft weiterhin ein Verfahren zur Herstellung des erfindungsgemäßen Adsorbergranulates. Die erfindungsgemäßen Adsorbergranulate sind über ein Suspensions-polymerisationsverfahren erhältlich, das folgende Schritte umfasst:

1. Suspendieren der Aktivkohlepartikel in Wasser, optional kann ein Stabilisator wie beispielsweise Polyvinylpyrrolidon oder Methacrylat zugegeben werden,
2. Zutropfen einer Monomerlösung enthaltend einen Radikalstarter, wie beispielsweise Azo-bis-(isobutyronitril) (AIBN), und einem Quervernetzer, wobei der Quervernetzer Ethylenglycoldimethacrylat umfasst, bei einer Temperatur von etwa 40 °C,
3. 2 - 3 h Rühren,
4. Aufheizen der Reaktionsmischung auf ca. 65 °C zur thermischen Aktivierung des Radikalstarters,
5. 10 - 12 h Rühren, beispielsweise über Nacht,
6. Ablassen der Reaktionsmischung über ein Sieb oder Sedimentieren der größeren Partikel und Dekantieren der kleineren Partikel,
7. Nachwaschen mit Wasser, und
8. Abfüllen des beschichteten Adsorbergranulates in feuchtem Zustand.

In einer bevorzugten Ausführungsform der vorliegenden Erfindung werden die Aktivkohlepartikel in einer Lösung suspendiert, die 2 Gewichtsprozent Polyvinylpyrrolidon enthält. Die Monomerlösung umfasst 1-Vinyl-2-pyrrolidon und der Quervernetzer umfasst Ethylenglykoldimethacrylat.

Das erfindungsgemäße Adsorbergranulat eignet sich zur Verwendung in der Nieren- oder Leberersatztherapie sowie in der Entgiftung und zur Behandlung einer Sepsis.

Die vorliegende Erfindung betrifft weiterhin eine Adsorberkartusche enthaltend das oben beschriebene hämokompatible Adsorbergranulat nach einem der Ansprüche 1 bis 4, sowie deren Verwendung in Vorrichtungen zur Hämoperfusion oder Plasmapherese. Die Adsorberkartusche ist in drei Teilbereiche eingeteilt ist: einen mittleren Bereich, welcher das Adsorbergranulat enthält, einen oberen Teilbereich mit dem Zuleitkanal für das Blut/Plasma sowie einen unteren Teilbereich mit dem entsprechenden Ableitkanal. Innerhalb der Kartusche dienen Trennelemente (z.B. Siebe) dazu, das Absorbergranulat im mittleren Bereich zurück zu halten. Die Trennelemente müssen so gestaltet sein, dass sie Blutzellen ohne Erzeugung von Scherstress durchlassen und gleichzeitig das Adsorbergranulat zurück halten. Die Durchlassweite der Trennelemente (Maschenweite oder Porenweite) liegt idealer Weise im Bereich von 40 bis 60 $\mu$m.

Die maximal mögliche Blut-/Plasmaflussrate steht in unmittelbarem Zusammenhang mit der Querschnittsfläche des mittleren Bereichs der Adsorberkartusche. Sie kann im Bereich von 10 cm$^2$ und 120 cm$^2$, vorzugsweise 20 cm$^2$ bis 80 cm$^2$, sein. Das Volumen des Absorbergranulats kann zwischen 30 und 1200 ml liegen.

Die Konnektoren der des Zuleit- und Ableitkanals müssen so gewählt werden, dass sie zu gängigen Vorrichtungen zur Hämoperfusion oder Plasmapherese passen, z.B. Luer-Verbindungen.

Die Adsorberkartusche und das Adsorbermaterial muss so beschaffen sein, dass es sterilisiert werden kann. Gängige Verfahren zur Sterilisation medizinischer Geräte sind die Sterilisation mit feuchter Hitze, in-line Dampfsterilisation oder Autoklavieren, sowie das Bestrahlen mit energiereicher, ionisierender Strahlung wie beispielsweise UV-Licht, Röntgenstrahlung, oder ionisierender Strahlung wie Alpha-, Beta oder Gamma -Strahlung.

Sobald Blut mit bioinkompatiblen Oberflächen in Kontakt kommt, kommt es zu einer Reaktion des Gerinnungssystems, des Komplementsystem und weiterer humoraler Faktoren wie beispielsweise Antikörper und Cytokine. Darüber hinaus haben die Oberflächen Einfluss auf das Blutbild.

Dem Fachmann sind Verfahren zur Beurteilung der Hämokompatibilität von mit Blut in Kontakt kommenden Oberflächen und geeignete Messparameter bekannt. Solche Untersuchungen können als Batch-Versuche in Probenröhrchen oder kontinuierlich im Rezirkulationsmodell durchgeführt werden. Auf diese Weise können die Hämokompatibilitätseigenschaften mit und ohne Einfluss der Durchströmung auf das Blut untersucht werden.

Ein Verfahren ist die Bestimmung der Plasmakonzentration des TAT-III-Komplexes. Antithrombin III inaktiviert hauptsächlich Thrombin, indem es durch die feste Bindung mit dem Serin im aktiven Zentrum der Proteasen einen irreversiblen Komplex mit denselben bildet. Bei der Neutralisierung des in freier Form praktisch nicht im Plasma vorkommenden Thrombins entsteht der sehr stabile TAT-III-Komplex im Verhältnis 1:1 mit einer Halbwertszeit von 10-15 Minuten. Im Normalfall beträgt die Plasmakonzentration dieses Komplexes weniger als 5 $\mu$g/l. Die Erfassung einer Thrombinbildung und damit der Nachweis eines ablaufenden Gerinnungsprozesses lassen sich anhand der Plasmakonzentration des TAT-III-Komplexes ermitteln. Aufgrund dieser Tatsachen wird der TAT-III-Komplex als indirektes Maß für die Bildung von Thrombin angesehen und reflektiert somit das Ausmaß einer Gerinnungsaktivierung.

Eine Aktivierung des Komplementsystems kann über die Konzentration der Komplementkomponente C5a nachgewiesen werden.

Die Bestimmung der C5a- und TAT-Werte erfolgt durch immunologische Nachweismethoden (ELISA).

Die Hämolyse kann über die Bestimmung der Konzentration von freiem Hämoglobin (fHb) und das Blutbild nachgewiesen werden. Zu den Blutbildparametern gehören die Zellzahlen von Thrombozyten (PLT), Erythrozyten (RBC) und Leukozyten (WBC), der Hämatokrit (HCT) und das Hämoglobin (HGB).

**Beispiele**

Für die Herstellung der erfindungsgemäßen Adsorbergranulate und der Vergleichsbeispiele wurden zwei Typen von Kugelaktivkohle mit einem mittleren Teilchendurchmesser von 510 $\mu$m bzw. 200 bis 400 $\mu$m verwendet. Die spezifische Oberfläche betrug 1433 m$^2$/g (gemessen über Stickstoffadsorption, BET-Methode). Das Porenvolumen (gemessen über Stickstoffadsorption, V-t, BJH) betrug: Mikroporen 0,69cm$^3$/g und Mesoporen 0,71cm$^3$/g.

**Beispiel 1: Herstellung eines PVP-beschichteten Adsorbergranulats**

In einem Glasreaktor (V = 5 l) mit Wendelrührer wurden 50 g Aktivkohle in 4 l einer 2%igen PVP-Lösung unter Rühren suspendiert und auf eine Temperatur von 40 °C erwärmt. Zu dieser Suspension wurden unter Rühren eine Lösung von 0,7 g AIBN, 80 g 1-Vinyl-2-pyrrolidon, 15 g Ethylenglykoldimethacrylat zugetropft. Die Reaktionsmischung wurde bei 40 °C für ca. 2 h weiter gerührt. Anschließend wurde die Reaktionsmischung auf 65 °C erhitzt und über Nacht weiter gerührt.

Danach wurde die Reaktionsmischung aus dem Reaktor in ein Sieb abgelassen und das erhaltene Granulat mit Wasser nachgewaschen. Das gewaschene Granulat wurde ohne weitere Trocknung in feuchtem Zustand in Weithalsflaschen abgefüllt.

**Beispiele 2 bis 6**

Analog zum in Beispiel 1 dargestellten Vorgehen wurden weitere Proben hergestellt. Die jeweiligen Zusammensetzungen sind in Tabelle 1 gegenübergestellt.

**Tabelle 1**

| Beispiel | 1 | 2 | 3 | 4 | 5 | 6 |
|---|---|---|---|---|---|---|
| Aktivkohle (g) | 50 | 50 | 50 | 50 | 50 | 50 |
| AIBN (g) | 0,7 | 0,7 | 0,7 | 0,7 | 0,7 | 0,7 |
| Vinylpyrrolidon (g) | 80 | 150 | 180 | 200 | 240 | 300 |
| Vinylpyrrolidon (mol) | 0,721 | 1,351 | 1,622 | 1,802 | 2,162 | 2,703 |
| Ethylenglycoldimethacrylat (g) | 15 | 30 | 72 | 80 | 99 | 99 |
| Ethylenglycoldimethacrylat (mol) | 0,076 | 0,151 | 0,363 | 0,404 | 0,499 | 0,499 |
| Monomer-Quervernet zer-Verhältnis $m$ | 90,5 | 89,9 | 81,7 | 81,7 | 81,2 | 84,4 |
| 2% Polyvinypyrrolidon in Wasser (Liter) | 4 | 4 | 4 | 4 | 4 | 4 |

Das Stoffmengenverhältnis m von Monomer zu Quervernetzer berechnet sich nach der Formel

$$m = \frac{n_{Monomer}}{(n_{Monomer} + n_{Quervernetzer})}$$

**Beispiel 7** nicht gemäß der Erfindung

In einem Glasreaktor (V = 5 l) mit Wendelrührer werden 50 g Aktivkohle in 4 l einer 2%igen PVP-Lösung unter Rühren suspendiert und auf eine Temperatur von 40 °C erwärmt. Zu dieser Suspension werden unter Rühren eine Lösung von 0,8 g AIBN in 180 g Glycidylmethacrylat und 72 g Ethylenglykoldimethacrylat zugetropft. Die Reaktionsmischung wurde bei 40 °C für ca. 2 h weiter gerührt. Anschließend wurde die Reaktionsmischung auf 65 °C erhitzt und über Nacht weiter gerührt.

Danach wurde die Reaktionsmischung aus dem Reaktor in ein Sieb abgelassen und das erhaltene Granulat mit Wasser nachgewaschen.

Das Glycidylmethacrylat wird mit 1 l 0,5 M Schwefelsäure 3h bei 50°C verseift und danach abgesaugt und neutral gewaschen.

Das gewaschene Granulat wurde ohne weitere Trocknung in feuchtem Zustand in Weithalsflaschen abgefüllt.

**Beispiel 8 Adsorption**

Von den in den Beispielen 1 bis 6 synthetisierten Adsorbermaterialen wurde die Adsorptionskapazität für verschiedene urämische Toxine bestimmt. Hierfür wurde jeweils 2,5 ml Adsorbergranulat in eine Säule (8x50 mm) gefüllt und mit einem Modellplasma (50 mg/ml BSA, 0,9 Gew.-% NaCl, 2,5 mmol/l CaCl2, 10 mmol/l K2HPO4 (pH 7,4) in bidestilliertes H2O, Fluss 0,2 ml/min) durchströmt.

In den Eingangsstrom wurde jeweils ein Toxin solange in Portionen von 100 μL injiziert bis die injizierte Masse ungefähr der Konzentration im Blut eines urämischen Patienten entsprach. Die Analyse des Modellplasmas am Ausgang der Säule erfolgte UV-spektrometrisch.

So lange keine Toxine im Ausgangstrom der Säule nachweisbar sind, wird das jeweilige Toxin vollständig vom Adsorbergranulat adsorbiert. Die Ergebnisse der Adsorptionsversuche sind in Tabelle 2 zusammengefasst. Alle sechs Proben adsorbierten die untersuchten urämischen Toxine vollständig.

In weiteren Versuchen konnte gezeigt werden, dass höhermolekulare Verbindungen wie Albumin und Heparin gar nicht bzw. nur in geringem Maß adsorbiert werden. Darüber hinaus wurde gefunden, dass sich das Adsorbergranulat der vorliegenden Erfindung sehr gut zur Adsorption von Bilirubin eignet. Bilirubin ist ein Hämoglobin Abbauprodukt, das an das Serumprotein Albumin gebunden vorliegt und bei Gesunden über die Leber ausgeschieden wird. Bei leberinsuffizienten Patienten ist die Bilirubinkonzentration im Plasma stark erhöht. Ziel einer Leberersatztherapie ist die Entfernung von Bilirubin aus dem Plasma durch Adsorption. Damit eignet sich das erfindungsgemäße Adsorbergranulat zur Verwendung in der Leberersatztherapie.

**Tabelle 2 Adsorption**

| Beispiel | injizierte Masse (mg) | 1 | 2 | 3 | 4 | 5 | 6 |
|---|---|---|---|---|---|---|---|
| Albumin | | 3 | 20 | 0 | 0 | 0 | 6 |
| Phenylessigsäure | 33,1 | 99 | 89 | 100 | 100 | 99 | 99 |
| Hydroxyhippursäure | 2,6 | 100 | 98 | 99 | 99 | 100 | 99 |
| Hippursäure | 18,0 | 99 | 98 | 98 | 98 | 99 | 97 |
| Indoxylsulfat | 4,0 | 99 | 100 | 100 | 99 | 99 | 100 |
| *p*-Cresylsulfat | 10,4 | 99 | - | 99 | 99 | 98 | 98 |
| Ap3A | | 98 | 94 | 91 | 87 | 99 | 94 |
| Ap5A | | 96 | 92 | 62 | 80 | 98 | 92 |
| Bilirubin | | 99 | 95 | 84 | 83 | 98 | 95 |
| Heparin | | 47 | 29 | 33 | 14 | 17 | 10 |

**Beispiel 9 Untersuchung der Hämokompatibilität**

Für die Untersuchung der Hämokompatibilität im Batch-Versuch werden zwei Zentrifugenröhrchen (V=50 ml) mit 2,5 ml des zu untersuchenden Materials und als Vergleichsprobe Adsorbergranulat aus einem DALI (Direct Adsorptin of Lipoproteins)-LDL Adsorber der Fa. Fresenius Medical Care gefüllt. Die beiden Zentrifugen-röhrchen und ein drittes Röhrchen als Leerprobe werden mit je 22,5 ml Vollblut gefüllt und 2 h bei 21 °C auf einem Rollenmischer inkubiert.

Zur Antikoagulation werden in allen Versuchen 1.5 IU/ml an Heparin eingesetzt.

Zu Versuchsende werden die Proben entnommen und die Analytik erfolgt. Für die Untersuchung des Eluats werden 5 ml Probenvolumen verwendet. An den Proben wurde jeweils die Konzentration von freiem Hämoglobin (fHb), der Komplement-komponente C5a und des TAT-Komplexes gemessen sowie das Blutbild bestimmt.

Zur quantitativen Beurteilung der Hämokompatibilität wird die Veränderung der Analyseparameter im zeitlichen Verlauf bestimmt. Das zu testende Adsorbermaterial wird gegen zwei Referenzsysteme bestehend aus Granulat aus einem DALI-Adsorber (Firma Fresenius Medical Care, Bad Homburg) und Granulat aus einer Hämoperfusionskartusche vom Typ Adsorba 300 C (Firma Gambro, Lund) sowie 0,9 % ige Kochsalzlösung als Leerwert verwendet. Die Durchführung des Rezirkulationsversuches erfolgt in einem Versuchsaufbau bestehend aus Blutschlauchsystemen mit Adsorberkartuschen und Blutpumpen. Das Blut rezirkuliert in Blutschlauchsystemen (PVC-Schlauch, Innendurchmesser 3,1 mm), welche an sogenannte Boards angebracht und in einen Wärmeschrank (37°C) integriert sind. Im Probenreservoir befindet sich ein Rührer, sodass eine ständige Durchmischung des Blutes gewährleistet ist. Durch eine Blutpumpe (Schlauchlänge 0,2 m, Innendurchmesser 4 mm) wird das Blut bei einem Fluss von 12,6 ml/min über den arteriellen Zweig gefördert (Länge 1,4 m), gelangt durch den Adsorber und venös (Länge 0,7 m) wieder zurück in das Probenreservoir. In der jeweiligen Adsorberkartusche befinden sich 5 ml Adsorbermaterial bzw. isotonische Kochsalzlösung.

Das Gehäuse der Adsorberkartusche bildet eine 20 ml Spritze aus Polyethylen mit einem leicht konischen Ausgang und einem Luer-Anschluss. Der Durchmesser der Kartusche beträgt 19 mm und die Höhe 40 mm und wird durch einem Deckel aus Plexiglas abgeschlossen. Am Boden der Kartusche befindet sich ein Siebgewebe (Maschenweite 40 $\mu$m), sodass das Beadmaterial zurückgehalten wird und das Blut ungehindert durchströmen kann.

Nach Versuchsende werden die Systeme erneut mit Kochsalzlösung gespült und optisch auf Gerinnselbildung untersucht.

Die Probennahme erfolgt nach 15, 30, 60, 120, 180 min. An den Proben wird wie bei den Batch-Versuchen jeweils die Konzentration von freiem Hämoglobin (fHb), der Komplementkomponente C5a und des TAT-Komplexes gemessen sowie das Blutbild bestimmt. Zu den Blutbildparametern gehören die Zellzahlen von Thrombozyten (PLT), Erythrozyten (RBC) und Leukozyten (WBC), der Hämatokrit (HCT) und das Hämoglobin (HGB). Die Auswertung und Analyse der Blutbildparameter und des freien Hämoglobins erfolgt am Versuchstag. Für die Bestimmung von TAT und C5a werden Proben eingefroren und die Parameter zu einem späteren Zeitpunkt bestimmt.

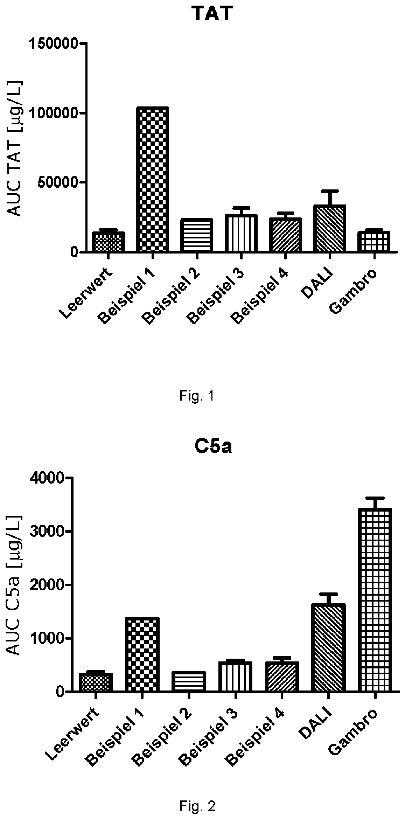

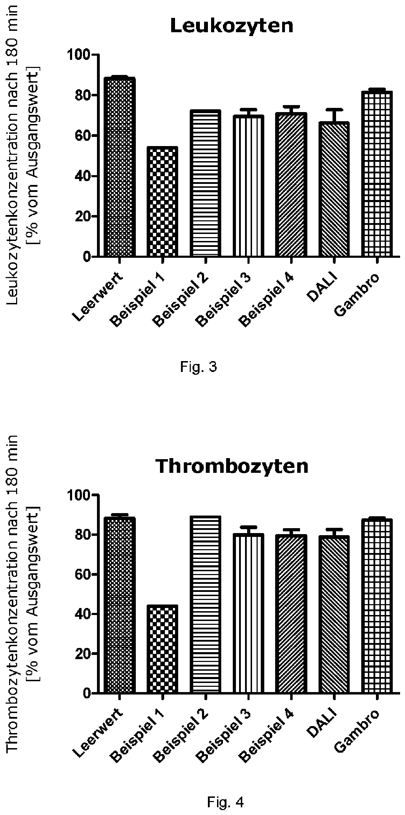

Die Ergebnisse der Versuche zur Hämokompatibilität sind in Tabelle 3 und den Figuren 1 bis 4 wiedergegeben.

**Tabelle 3**

| Beispiel | 1 | 2 | 3 | 4 | DALI | Adsorba 300 C |
|---|---|---|---|---|---|---|
| TAT ($\mu$g/L) | 103428 | 22994 | 26151 | 23778 | 32973 | 13819 |
| C5a ($\mu$g/L) | 1370 | 357 | 531 | 531 | 1621 | 3403 |
| Leukozyten* | 54 | 72 | 69 | 71 | 66 | 81 |
| Thrombozyten* | 44 | 89 | 80 | 80 | 79 | 87 |
| *)in % vom Ausgangswert nach 180 min | | | | | | |

**Patentansprüche**

1. Hämokompatibles Adsorbergranulat zur Entfernung proteingebundener Toxine aus Blut oder Blutplasma umfassend mit einer hydrophilen Polymerschicht überzogene sphärische Aktivkohlepartikel **dadurch gekennzeichnet, dass** die Polymerschicht quervernetzt ist und wobei das Polymer 1-Vinyl-2-pyrrolidon-Monomere und der Quervernetzer Ethylenglykoldimethacrylat umfasst.

2. Adsorbergranulat nach Anspruch 1, wobei das Polymer eine Schichtdicke von 50 nm bis 20 $\mu$m aufweist.

3. Adsorbergranulat nach einem der vorhergehenden Ansprüche, wobei das Adsorbergranulat eine Sphärizität von >0,9 aufweist.

4. Adsorbergranulat nach einem der vorhergehenden Ansprüche, wobei das Adsorbergranulat eine mittlere Partikelgröße von 100 - 1000 $\mu$m aufweist.

5. Adsorbergranulat nach einem der vorhergehenden Ansprüche zur Verwendung in der Nierenersatztherapie.

6. Adsorbergranulat nach einem der vorhergehenden Ansprüche zur Verwendung in der Leberersatztherapie.

7. Adsorbergranulat nach einem der vorhergehenden Ansprüche zur Verwendung in der Behandlung von Sepsis.

8. Adsorbergranulat nach einem der vorhergehenden Ansprüche zur Verwendung in der Behandlung von Vergiftungen.

9. Verfahren zur Herstellung eines Adsorbergranulates nach einem der Ansprüche 1 bis 4 umfassend die Schritte:

   a. Suspendieren von Aktivkohlepartikeln in Wasser,
   b. Zugabe der Monomere, des Quervernetzers und eines Radikalstarters,
   c. Erhitzen der Reaktionsmischung,
   d. Abtrennen der beschichteten Partikel.

10. Adsorberkartusche enthaltend ein hämokompatibles Adsorbergranulat nach einem der Ansprüche 1 bis 4.

**Claims**

1. Hemocompatible adsorber granules for removing protein-bound toxins from blood or blood plasma, comprising spherical activated carbon particles coated with a hydrophilic polymer layer, **characterized in that** the polymer layer is crosslinked, and wherein the polymer comprises 1-vinyl-2-pyrrolidone monomer and the crosslinking agent is ethylene glycol dimethacrylate.

2. The adsorber granules according to claim 1, wherein the polymer has a layer thickness of 50 nm to 20 $\mu$m.

3. The adsorber granules according to any one of the preceding claims, wherein the adsorber granules have a sphericity of >0.9.

**4.** The adsorber granules according to any one of the preceding claims, wherein the adsorber granules have an average particle size of 100-1000 $\mu$m.

**5.** The adsorber granules according to any one of the preceding claims, for use in renal replacement therapy.

**6.** The adsorber granules according to any one of the preceding claims, for use in liver replacement therapy.

**7.** The adsorber granules according to any one of the preceding claims, for use in the treatment of sepsis.

**8.** The adsorber granules according to any one of the preceding claims, for use in the treatment of toxicities.

**9.** A method for producing adsorber granules according to any one of claims 1 to 4, comprising the steps:

    a. suspending activated carbon particles in water,
    b. adding the monomers, crosslinking agent and a radical initiator,
    c. heating the reaction mixture,
    d. separating the coated particles.

**10.** The adsorber cartridge containing hemocompatible adsorber granules according to any one of claims 1 to 4.

**Revendications**

**1.** Granulés adsorbeurs hémocompatibles pour l'élimination des toxines liées aux protéines du sang ou du plasma sanguin, comprenant des particules sphériques de charbon actif revêtues d'une couche de polymère hydrophile, **caractérisés en ce que** la couche de polymère est réticulée transversalement, le polymère comprenant des monomères de 1-vinyle-2-pyrrolidone et l'agent de réticulation transversale comprenant de l'éthylène glycol diméthacrylate.

**2.** Granulés adsorbeurs selon la revendication 1, dans lesquels le polymère présente une épaisseur de couche de 50 nm à 20 $\mu$m.

**3.** Granulés adsorbeurs selon une des revendications précédentes, les granulés adsorbeurs présentant une sphéricité > 0,9.

**4.** Granulés adsorbeurs selon une des revendications précédentes, les granulés adsorbeurs présentant une taille moyenne de particules de 100 à 1000 $\mu$m.

**5.** Granulés adsorbeurs selon une des revendications précédentes, destinés à une utilisation en thérapie de substitution rénale.

**6.** Granulés adsorbeurs selon une des revendications précédentes, destinés à une utilisation en thérapie de substitution hépatique.

**7.** Granulés adsorbeurs selon une des revendications précédentes, destinés à une utilisation dans le traitement de la septicémie.

**8.** Granulés adsorbeurs selon une des revendications précédentes, destinés à une utilisation dans le traitement des intoxications.

**9.** Procédé de fabrication de granulés adsorbeurs selon une des revendications 1 à 4, comprenant les étapes suivantes :

    a. mise en suspension de particules de charbon actif dans de l'eau,
    b. ajout des monomères de l'agent de réticulation transversale et d'un initiateur radicalaire,
    c. chauffe du mélange réactionnel,
    d. isolation des particules revêtues.

**10.** Cartouche d'adsorbeur contenant des granulés adsorbeurs hémocompatibles selon une des revendications 1 à 4.

## TAT

Fig. 1

## C5a

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013004604 A
- US 3983053 A
- DE 7425290 U
- US 4048064 A
- US 4169051 A
- US 2004001800 A
- FR 2148022
- FR 2239282

**In der Beschreibung aufgeführte Nicht-Patentliteratur**

- **R. VANHOLDER et al.** *Kidney International,* 2003, vol. 63, 1934-1943
- **LIABEUF et al.** *Nephrol Dial Transplant,* 2010, vol. 25, 1183-1191
- **BARRETO et al.** *Clin JAm Soc Nephrol,* 2009, vol. 4, 1551-1558